# EUROPEAN PATENT APPLICATION

(11) **EP 3 287 017 A1**
(43) Date of publication of application: **28.02.2018**
(21) Application number: 15889604.3
(22) Date of filing: 15.06.2015
(51) Int. Cl.: A24F 47/00

(54) **ATOMIZER AND AEROSOL GENERATION DEVICE THEREOF**

(30) Priority: 22.04.2015 CN 201510192005
(71) Applicant: Joyetech (Changzhou) Electronics Co., Ltd., Changzhou, Jiangsu 213125 (CN)
(72) Inventor: QIU, Weihua, Changzhou Jiangsu 213125 (CN)
(74) Representative: CMS Cameron McKenna Nabarro Olswang LLP
(86) International application number: PCT/CN2015/081479
(87) International publication number: WO 2016/169115

(57) **Abstract**

An atomizer for an aerosol generating device includes a heating member and a liquid guiding member stacked in the heating member. Other atomizers which include a heating member and a liquid guiding member and which are of equal performance may have the liquid guiding member coated on, twined around, or stacked on the outer walls of the heating member. An aerosol generating device including any or all of the atomizers described above is also provided.

## Description

### FIELD

The subject matter herein generally relates to an aerosol generating device, and particularly to an atomizer and an aerosol generating device.

### BACKGROUND

An electronic cigarette, which is also known as a virtual cigarette, an electronic atomizer, and an electronic cigar, has a taste similar to a cigarette.

Currently, the electronic cigarettes on the market heat and atomize the smoke liquid through a heating member which twines around a liquid guiding member.

### SUMMARY OF THE INVENTION

The present disclosure relates to an atomizer and an aerosol generating device including the atomizer.

An atomizer includes a heating member and a liquid guiding member disposed in the heating member.

In an alternative embodiment, the liquid guiding member is a porous permeable member which is in fluid communication with a liquid storage device through a liquid guiding hole defined on the heating member.

In an alternative embodiment, the heating member is a tubular member with an opening, and the liquid guiding member contacts the inner walls of the heating member.

In an alternative embodiment, the heating member is reticular, that is, the heating member defines a number of through holes. A number of heating wires are woven together to form the reticular heating member, or the reticular heating member are formed by defining the through holes on a heating plate. The through holes on the heating plate are formed by using laser pulses, by a computer numerical control (CNC) process, or by a punching process.

In an alternative embodiment, the heating member has properties of capillary action and/or super capillary action. The super capillary action can be realized by etching on the surface of the metal using high energy laser pulses. That is, the surface of the heating member defines a number of suction grooves and/or capillary channels.

In an alternative embodiment, when the heating member is made of metallic material, the suction grooves and/or capillary channels are formed by sintering metallic powder particles using a solid phase sintering method; when the heating member is made of ceramic materials (e.g., porous high temperature resistant ceramic), the heating member is formed by sintering ceramic powder particles using the solid phase sintering method.

In an alternative embodiment, a number of tiny heating wires are woven together into strips, and then the strips are woven into a net to form the heating member. Gaps between the tiny heating wires form the suction holes, thus the heating member has an absorbent property for smoke liquid.

In an alternative embodiment, the heating member and the liquid guiding member are the same one.

An atomizer includes a heating member and a liquid guiding member which is coated on, twined around, or stacked in the heating member.

In an alternative embodiment, the heating member includes at least one recess, and the liquid guiding member is partially received in the at least one recess.

In an alternative embodiment, the at least one recess is formed by folding and bending the heating plate or by cutting the heating plate.

In an alternative embodiment, the heating member is sandwiched between two liquid guiding members.

In an alternative embodiment, the heating member defines a number of suction grooves and/or capillary channels.

An aerosol generating device includes the atomizer described above.

The present disclosure includes the following advantages:

First, the atomizer and the aerosol generating device have a simple structure, thus it benefits the manufacture of the atomizer and the aerosol generating device.

Second, because of the through holes on the heating member, the heating member avoids dry heating, and the smoke liquid (aerosol matrix) flows out conveniently after being atomized.

Third, because the heating member has the properties of capillary action, the suction grooves and/or the capillary channels defined on the heating member can store smoke liquid to keep the walls of the heating member sufficiently wet. In the heating process, the heating member atomizes the smoke liquid stored in the suction grooves and/or the capillary channels. This can prevent the heating element from dry heating and affecting the user's suction feeling, and also can prevent the heating member from being ashed and burning by overheating. Thus, the health of the users can be ensured.

### BRIEF DESCRIPTION OF THE DRAWINGS

Implementations of the present technology will now be described, by way of example only, with reference to the attached figures.
FIG. 1 is a cross-section view, taken along an axis direction of a first exemplary embodiment of a portion of an atomizer.
FIG. 2 is a cross-section view, taken along a radius direction of the atomizer of FIG. 1.
FIG. 3 is an enlarged view of a part of the heating member of FIG.1.
FIG. 4 is a schematic diagram of the first exemplary embodiment of an aerosol generating device.
FIG. 5 is a cross-section view of a second exemplary embodiment of an atomizer.
FIG. 6 is an isometric view of a cylindrical spiral atomizer of FIG. 5.
FIG. 7 is an isometric view of a conical spiral atomizer of FIG. 5.
FIG. 8 is an isometric view of a square cylinder spiral atomizer of FIG. 5.
FIG. 9 is an isometric view of a pie shaped atomizer of FIG. 5.
FIG.10 is a schematic diagram of the second exemplary embodiment of an aerosol generating device.
FIG. 11 is a schematic diagram of a third exemplary embodiment of an atomizer.
FIG. 12 is a schematic diagram of a third exemplary embodiment of an aerosol generating device.
FIG. 13 is a schematic diagram of a fourth exemplary embodiment of an atomizer.

In the attached figures, atomizers are labeled 10, 20, 30, and 40; atomizing devices are labeled 140, 240, 340; heating members are labeled 12, 22, 32, and 42; air pipe is labeled 160; liquid guiding members are labeled 14, 24, 34, and 44; liquid storage device is labeled 180; through hole is labeled 122; suction device is labeled 190; suction groove is labeled 124; atomizing chambers are labeled 141, 241, and 341; capillary channel is labeled 126; recess is labeled 322; control device is labeled 170; battery device is labeled 120; aerosol generating devices are labeled 100, 200, 300, and 400.

### DETAILED DESCRIPTION

It will be appreciated that for simplicity and clarity of illustration, where appropriate, reference numerals have been repeated among the different figures to indicate corresponding or analogous elements. In addition, numerous specific details are set forth in order to provide a thorough understanding of the embodiments described herein. However, it will be understood by those of ordinary skill in the art that the embodiments described herein can be practiced without these specific details. In other instances, methods, procedures, and components have not been described in detail so as not to obscure the related relevant feature being described. Also, the description is not to be considered as limiting the scope of the embodiments described herein. The drawings are not necessarily to scale and the proportions of certain parts may be exaggerated to better illustrate details and features of the present disclosure.

### First Exemplary Embodiment:

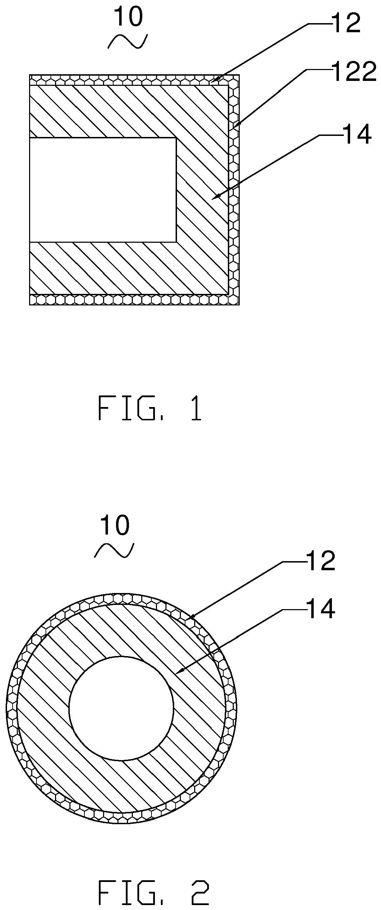

FIGS. 1-2 illustrate a portion of an atomizer 10 which includes a heating member 12 and a liquid guiding member 14 positioned in the heating member 12. In the illustrated embodiment, the heating member 12 can be a tubular member with an opening. The liquid guiding member 14 can be situated in the heating member 12 and be in contact with the inner walls of the heating member 12. The liquid guiding member 14 can be a porous permeable member extending through a liquid guiding hole defined in the heating member 12, to fluidly communicate with a liquid storage device.

The heating member 12 can be reticular. That is, the heating member 12 can be a mesh configuration defining a number of through holes 122. A number of heating wires are woven together to form the heating member 12 or a number of through holes 122 are defined in a heating plate to form the heating member 12. The through holes 122 of the heating plate can be formed by using laser pulses, by a computer numerical control (CNC) process, or by a punching process. The heating member 12 can be made of conductive metal, such as stainless steel or nickel. In different embodiment, different electrical resistances of the heating member can be obtained by adjusting the size of the through holes 122, the diameter of the of the heating wires, the length of the heating wires, and other parameters. The shape of the through holes 122 is not limited to a circular shape. In an alternative embodiment, the through holes 122 can be square, petal shaped, star shaped, and so on. Because of the through holes 122, the smoke liquid (aerosol matrix) can flow out conveniently after atomization.

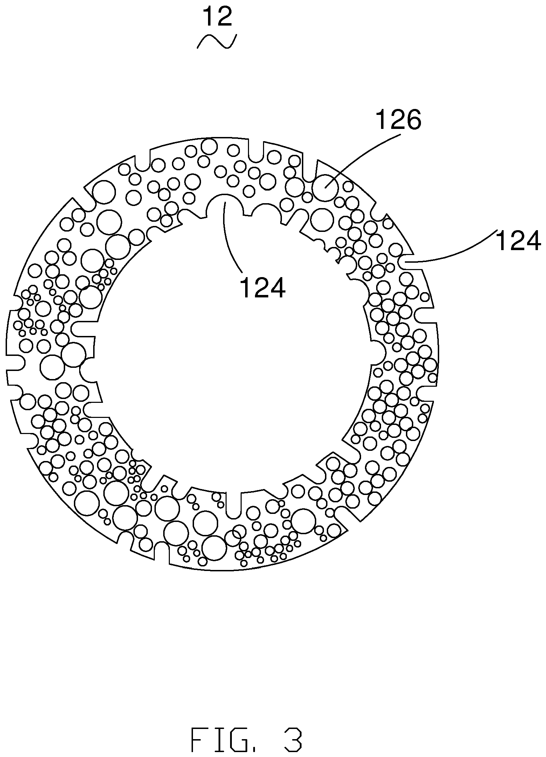

FIG. 3 illustrates that the heating member 12 can have properties of exciting capillary action and super capillary action. In detail, the capillary action can be realized by setting parameters such as the size of the through holes 122 and the thickness of the heating member 12. The super capillary action can be realized by etching on the surface of the metal using high energy laser pulses. That is, the surface of the heating member 12 can define a number of suction grooves 124 and/or capillary channels 126. Because the heating member 12 has the property of enabling capillary action, the suction grooves 124 and/or the capillary channels 126 defined in the heating member 12 can be configured to store smoke liquid to keep the walls of the heating member 12 sufficiently wet. In the heating process, the heating member 12 can atomize the smoke liquid stored in the suction grooves 124 and/or the capillary channels 126. This can prevent the heating element from dry heating and affecting the user's suction feeling, and also can prevent the heating member from being ashed and burning by overheating. Thus, the health of the users can be ensured.

In an alternative embodiment, the heating member 12 can be made of metallic material. The suction grooves 124 and/or the capillary channels 126 can be formed by sintering the metal powder particles using a solid phase sintering method. If the heating member 12 is made of ceramic materials, such as porous high temperature resistant ceramic, the heating member 12 can be formed by sintering ceramic powder particles using the solid phase sintering method.

In an alternative embodiment, a number of tiny heating wires can be woven together into strips, and then the strips can be woven into a net to form the heating member 12. Gaps between the tiny heating wires can form the suction holes, thus the heating member 12 can have an absorbent property for smoke liquid.

In an alternative embodiment, the heating member 12 is not limited to being a plate structure, the heating member 12 can be tubular.

In an alternative embodiment, the heating member 12 and the liquid guiding member 14 can be the same one.

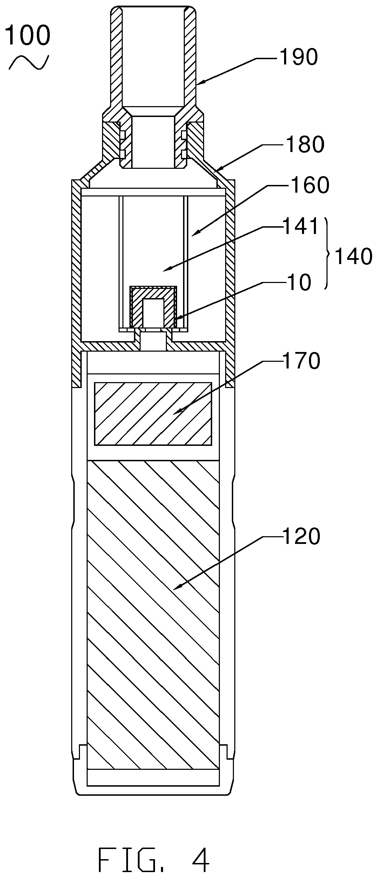

FIG. 4 illustrates an aerosol generating device 100 including the atomizer 10. The aerosol generating device 100 can include a battery device 120, an atomizing device 140, an air pipe 160, a liquid storage device 180, and a suction device 190. The battery device 120 can be electrically coupled to the atomizing device 140, and configured to supply power to the atomizing device 140. The atomizing device 140 can include the atomizer 10 and an atomizing chamber 141 in which the atomizer 10 is positioned. The aerosol generating device 100 can further include a control device 170 which is electrically coupled to the battery device 120 and the atomizing device 140.

### Second Exemplary Embodiment:

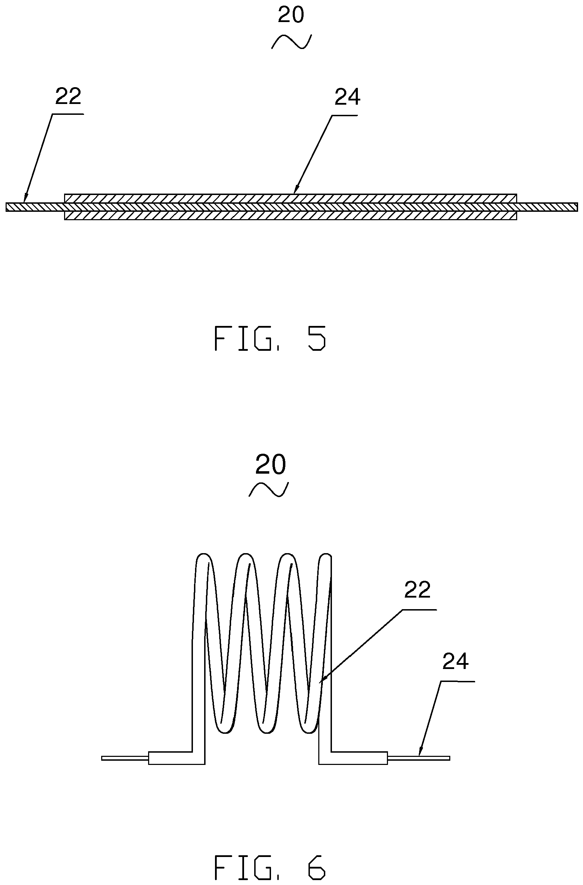

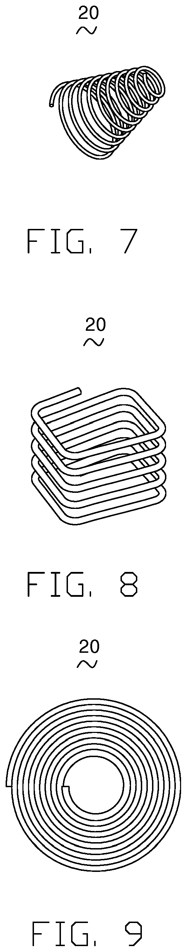

FIGS. 5-6 illustrate an atomizer 20, which includes a heating member 22 and a liquid guiding member 24. The liquid guiding member 24 can be coated on, twined around, or stacked on the outer walls of the heating member 22. FIGS. 7-9 illustrate that the atomizer 20 can be processed into a conical spiral structure, a square cylindrical spiral structure, and a pie or plate shaped structure, respectively, to satisfy different needs of atomizing device/aerosol generating device.

In an alternative embodiment, the heating member 22 can define the suction grooves 124 and/or the capillary channels 126.

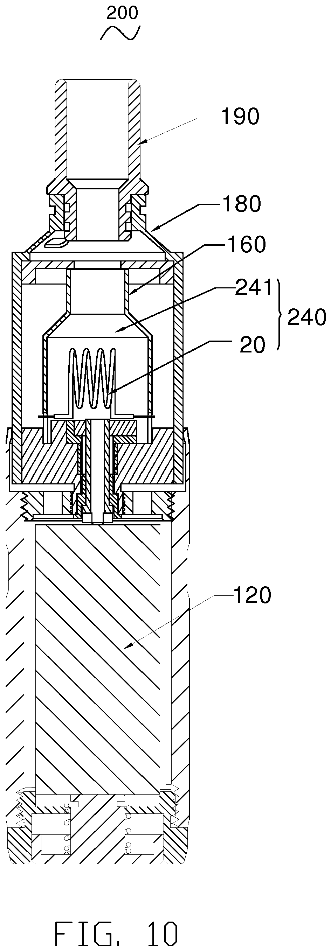

FIG. 10 illustrates an aerosol generating device 200 which includes the atomizer 20. The aerosol generating device 200 can include a battery device 120, an atomizing device 240, an air pipe 160, a liquid storage device 180, and a suction device 190. The battery device 120 can be electrically coupled to the atomizing device 240, and configured to supply power to the atomizing device 240. The atomizing device 240 can include the atomizer 20 and an atomizing chamber 241 in which the atomizer 20 is positioned. The aerosol generating device 200 can further include a control device 170 electrically coupled to the battery device 120 and the atomizing device 240.

### Third Exemplary Embodiment:

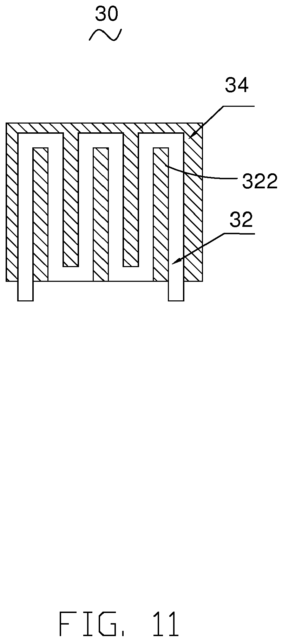

FIG. 11 illustrates an atomizer 30 which includes a heating member 32 and a liquid guiding member 34 coated on the outer walls of the heating member 32. In the illustrated embodiment, the heating member 32 can include at least one recess 322. The liquid guiding member 34 can be partially received in the at least one recess 322. The at least one recess 322 can be formed by folding and bending the heating plate or by cutting the heating plate.

In an alternative embodiment, the heating member 32 can be a number of heating wires, a number of heating rods, a reticular heating plate, or a mesh woven by heating wires, so long as at least a part of the heating member 32 can be embedded or inserted into the liquid guiding member 34.

In an alternative embodiment, the heating member 32 also can define the suction grooves 124 and/or the capillary channels 126.

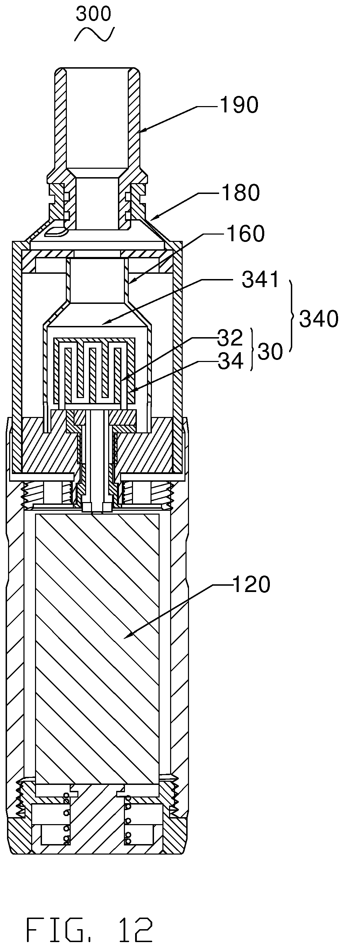

FIG. 12 illustrates an aerosol generating device 300, which includes the atomizer 30. The aerosol generating device 300 can include a battery device 120, an atomizing device 340, an air pipe 160, a liquid storage device 180, and a suction device 190. The battery device 120 can be electrically coupled to the atomizing device 340, and configured to supply power to the atomizing device 340. The atomizing device 340 can include the atomizer 30 and an atomizing chamber 341 in which the atomizer 30 is positioned. The aerosol generating device 300 can further include a control device 170 electrically coupled to the battery device 120 and the atomizing device 340.

### Fourth Exemplary Embodiment:

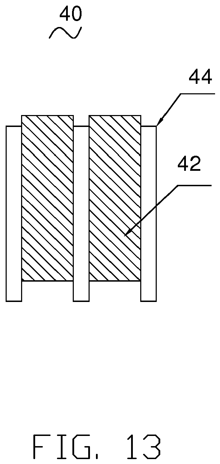

FIG. 13 illustrates an atomizer 40 which includes a heating member 42, and at least two liquid guiding members 44 stacked with the heating member 42. One heating member 42 can be sandwiched between two liquid guiding members 44.

In an alternative embodiment, the heating member 42 also can define the suction grooves 124 and/or the capillary channels 126.

The illustrated embodiment can also provide an aerosol generating device including the atomizer 40.

In an alternative embodiment, the atomizers 10, 20, 30, and 40 can be applied to other atomizing devices which require other atomizing methods, such as a spray atomizing device.

The technical characteristics of the above embodiments can be arbitrarily combined. In order to simplify the description, various characteristics of the various combinations of the above embodiments are not described. However, so long as there is no contradiction in combinations of the technical characteristics, such combinations are within the scope of this specification.

The embodiments shown and described above are only examples. Many details in this field are found in the art. Therefore, many such details are neither shown nor described. Even though numerous characteristics and advantages of the present technology have been set forth in the foregoing description, together with details of the structure and function of the present disclosure, the disclosure is illustrative only, and changes may be made in the detail, especially in matters of shape, size, and arrangement of the parts within the principles of the present disclosure, up to and including the full extent established by the broad general meaning of the terms used in the claims. Therefore, those of ordinary skill in the art can make various modifications to the embodiments without departing from the scope of the disclosure, as defined by the appended claims.

## Claims

1. An atomizer comprising:
a heating member; and
a liquid guiding member stacked in the heating member.

2. The atomizer of claim 1, wherein the liquid guiding member is a porous permeable member, and in fluidic communication with a liquid storage device through a liquid guiding hole defined on the heating member.

3. The atomizer of claim 1, wherein the heating member is a tubular member with an opening, and the liquid guiding member contacts the inner walls of the heating member.

4. The atomizer of claim 1, wherein the heating member defines a number of through holes.

5. The atomizer of any one of claims 1-4, wherein the heating member has properties of capillary action and/or super capillary action, a surface of the heating member defines a number of suction grooves and/or capillary channels.

6. An atomizer comprising:
a heating member; and
a liquid guiding member coated on, twined around, or stacked on the outer walls of the heating member.

7. The atomizer of claim 6, wherein the heating member comprises at least one recess, the liquid guiding member is partially received in the at least one recess.

8. The atomizer of claim 6, wherein the atomizer comprises at least two liquid guiding members, and the heating member is sandwiched between the at least two liquid guiding members.

9. The atomizer of claim 6, wherein the heating member defines a number of suction grooves and/or capillary channels

10. An aerosol generating device comprising the atomizer of any one of claims 1-9.
